# EUROPÄISCHE PATENTSCHRIFT

(11) **EP 0 481 240 B1**
(45) Veröffentlichungstag und Bekanntmachung des Hinweises auf die Patenterteilung: **11.12.1996**
(21) Anmeldenummer: 91116011.7
(22) Anmeldetag: 20.09.1991
(51) Int. Cl.: C08B 37/14, A61K 47/36

(54) **Galactomannanderivate zur Umhüllung oder Einbettung von Arzneimittelwirkstoffen**
Galactomannan derivate for the encapsulation or incorporation of drugs
Dérivé de galactomannane pour l'encapsulation ou l'incorporation de médicaments

(30) Priorität: 18.10.1990 DE 4033041
(43) Veröffentlichungstag der Anmeldung: 22.04.1992
(73) Patentinhaber: BASF AKTIENGESELLSCHAFT, 67056 Ludwigshafen (DE)
(72) Erfinder: Bauer, Kurt Heinz, Prof. Dr., W-7800 Freiburg 33 (DE); Wohlschlegel, Christian, Dr., W-5063 Overath (DE); Sarlikiotis, Antonis, W-7800 Freiburg (DE)

(56) Entgegenhaltungen:
- DE-A- 1 617 626
- FR-A- 2 143 059
- GB-A- 498 149
- NATURAL PLANT HYDROCOLLOIDS; ADVANCES IN CHEMISTRY SERIES Nr. 11, 1954, Seiten 51 - 61 H. DEUEL & H. NEUKOM 'Some properties of Locust Bean Gum'
- POLYMER PREPRINTS Bd. 30, Nr. 1, April 1989, US Seiten 480 - 481 C.M. LANCASTER & M.A. WHEATLEY 'Drug delivery to the colon: polymer susceptibility to degradation by colon contents'
- CHEMICAL ABSTRACTS, vol. 101, 1984, Columbus, Ohio, US; abstract no. 60033b, 'Carboxymethylated galactomannan products as pharmaceutical adjuvants' Seite 298 ;Spalte R ;

## Beschreibung

Es ist bekannt hydrophile Polymere, beispielsweise Hydroxypropylmethylcellulose oder auch Carboxymethylgalactomannane zur Herstellung von nichtmedizinischen Zubereitungen, die antimikrobische Wirkstoffe enthalten, herzustellen (Europa-Anmeldung Nr. 285 209).
Hydroxyalkylether von Polygalactomannanen sind weiterhin als Stabilisatoren für Kohle-, Wassermischungen (Europa-Anmeldung Nr. 209 122), sowie zur Klärung von Wasser (US-Patent Nr. 3 830 736) bekannt. Die US-Patentschrift Nr. 3 740 388 betrifft ein Herstellungsverfahren für Carboxyalkylether von Polygalactomannanen, wobei solche Reaktionsprodukte in Form von Komplexverbindungen mit Calcium als Verdickungsmittel verwendet werden können.

Weiterhin sind in der Literatur verschiedene Galactomannanester, sowie Galactomannanether beschrieben. Diese Galactomannanether werden jedoch nach sehr aufwendigen Verfahren gewonnen. Außerdem liegt der durchschnittliche Substitutionsgrad dieser bekannten Produkte zwischen 0,1 und 1,6, das heißt er ist erheblich niedriger als der Substitutionsgrad der erfindungsgemäßen Verbindung (siehe Bartl, H., Falbe, J., Houben-Weyl Methoden der organischen Chemie, Band E 20/3, Thieme, Stuttgart, 1987; Carson, J.F., Maclay, W.D., J. Am. Chem. Soc., 70, 1948, 293; Heyne, E., Whistler, R.L., J. Am. Chem. Soc., 70, 1948, 2249).

In der Stelle J. Am. Chem. Soc., 70, 1948, 2249 sowie der Stelle Swanson, J.W., J. Am. Chem. Soc., 71, 1949, 1511, werden zwar Methylgalactomannane erwähnt, mit höherem Substitutionsgrad, jedoch haben die so hergestellten Mannane ein niedrigeres Molekulargewicht als die erfindungsgemäßen, das heißt es handelt sich hierbei zwar um Methylgalactomannane, jedoch haben diese ein wesentlich niedrigeres Molekulargewicht als die erfindungsgemäßen Galactomannan-Derivate.

Aus Natural Plant Hydrocolloids; Advances in Chemistry Series, Nr. 11, 1954, S. 51-61, ist Johannisbrotkornmehl bekannt, dessen OH-Gruppen verestert oder verethert sein können. In Polymer Preprints Bd. 30, Nr. 1, April 1989, S. 480 - 481, wird beschrieben, daß bestimmte Polymere, darunter auch Galactomannane, durch physikalische oder chemische Vernetzung unlöslicher gemacht werden können.

Die vorliegende Erfindung betrifft die Verwendung von Galactomannanen, deren Hydroxygruppen vollständig oder teilweise in Form niedrigmolekularer aliphatischer, araliphatischer und/oder aromatischer Ether- oder Estergruppen vorliegen und 20 bis 80 %, vorzugsweise 30 bis 80 % beziehungsweise 30 - 70 %, insbesondere 40 bis 60 % oder auch 45 bis 55 % der vorhandenen Mannosebausteine der Galactomannan-Kette zusammenhängende Mannose-Blöcke von 2 bis 20, vorzugsweise 5 - 20 Mannose-Einheiten bilden, die nicht durch Galactose-Gruppen substituiert sind, für Umhüllungen und/oder Einbettungen von festen und/oder flüssigen Wirkstoffen, welche diese Wirkstoffe erst im Dickdarm oder in Gegenwart von glykolytischen Enzymen freigeben.
Vorzugsweise handelt es sich um Galactomannane, deren Ethergruppen aus C₁-C₆-Alkoxygruppen (insbesondere C₂-C₄-Alkoxygruppen), Phenyl-C₁-C₄-Alkoxygruppen oder Phenoxygruppen bestehen und worin die Estergruppen aus C₃-C₂₂-Alkanoyloxygruppen, vorzugsweise C₃-C₁₆-Alkanoyloxygruppen bestehen. Das Molgewicht der erfindungsgemäßen verwendeten Galactomannane liegt zwischen 10⁴ und 10⁸ insbesondere 10⁵ und 10⁶ g.
Das Mannose/Galactose-Verhältnis der zugrundeliegenden Galactomannan-Kette liegt statistisch beispielsweise zwischen 2 : 1 und 20 : 1 vorzugsweise zwischen 4 : 1 bis 10 : 1. Im Durchschnitt sind die freien Hydroxygruppen des hier zugrundeliegenden Galactomannans zu 80 bis 100 % verethert und/oder verestert.
Die gerade Grundkette der erfindungsgemäß verwendeten Galactomannane besteht aus Mannopyranose-Einheiten in 1.4-Verknüpfung. Die Galactose Moleküle sind glykosidisch mit dieser Kette über die CH₂-OH-Gruppen der Mannose-Glieder verknüpft.

Die erfindungsgemäß verwendeten Galactomannanderivate können beispielsweise zur Umhüllung oder Einbettung von Arzneiwirkstoffen oder auch zur Herstellung von Folien verwendet werden.

Es war überraschend, daß die erfindungsgemäß verwendeten Galactomannanderivate Wirkstoffe, die hierin eingebettet sind oder von solchen Galctomannderivaten umhüllt werden, erst im Dickdarm freigegeben werden, beziehungsweise erst in Gegenwart von glykolytischen Enzymen, nicht jedoch beispielsweise im Milieu des Magensaftes oder im Milieu des Dünndarms.

Die erfindungsgemäß verwendeten Galactomannanderivate sind also insbesondere zur Herstellung von Arzneimittelzubereitungen geeignet, wo die Wirkstoffe vor den Einflüssen des Magen- und Dünndarmsaftes geschützt werden müssen oder sollen und diese Wirkstoffe erst im Dickdarm freigesetzt werden sollen. Bisher mußten in solchen Fällen die entsprechenden Wirkstoffe in der Regel parenteral appliziert werden.

Bisher bekannte Arzneiformen zur Freisetzung von Wirkstoffen im Dickdarm, bei denen die Arzneistoffe oder Arzneistoffkerne mit Polymeren umhüllt oder in Polymere eingebettet waren, erreichen dieses Ziel nicht oder nur mangelhaft. Die hierfür verwendeten Filmüberzüge oder Einbettungsmaterialien bestehen aus herkömmlichen Filmbildnern, welche durch Vernetzung mit Azogruppen modifiziert sind (Saffran et al., U.S. Pat. 4 663 308) oder aus Mischungen derselben (Rubinstein et al., J. Pharm. 30, 95 bis 99 (1986)).

Die Arbeitsgruppe um Saffran polymerisierte Methacrylate mit verschiedenen Monomeren wie zum Beispiel Styrol und einer polymerisationsfähigen Azokomponente zum Beispiel Divinylazobenzol. Auf diese Weise sollten quervernetzte Polymere entstehen. Ein Abbau dieser Polymere im Colon konnte auch nicht innerhalb von 8 Tagen nachgewiesen werden. Für den vorgesehenen Verwendungszweck ist jedoch ein Abbau innerhalb von Minuten oder wenigen Stunden zu fordern. Außerdem sind primäre aromatische Amine als eventuelle Abbauprodukte toxikologisch bedenklich.

Die Arbeitsgruppe um Rubinstein verwendete Mischungen verschiedener Polymethacrylate, wobei die resultierenden Fllmüberzüge ihre Permeabilität oder Löslichkeit in Abhängigkeit des pH-Wertes ändern. Mit diesen Überzügen umhüllte oder in diese Polymere eingebettete Wirkstoffe können nur diffusionskontrolliert freigesetzt werden. Eine diffusionskontrollierte Freisetzung ist jedoch zu langsam, da die zur Verfügung stehende Freisetzung- und Resorptionszeit im Colon limitiert ist. Außerdem ist der Beginn der Freisetzung ungünstigerweise noch von einem Anstieg des pH-Wertes des Darminhaltes abhängig. In dem in Frage kommenden Darmabschnitt ist der pH-Wert jedoch bereits weitgehend konstant. Somit ergibt sich der Zeitpunkt des Freisetzungsbeginns aus der Transportgeschwindigkeit des Darminhaltes. Solche Filmüberzüge beginnen also bereits im proximalen oder im distalen Ileum zu quellen, um den Wirkstoff dann im Colon ascendens durch den maximal gequollenen Filmüberzug freizusetzen. Eine solche Arzneiform führt bestenfalls zu einer geringen und nicht reproduzierbaren Bioverfügbarkeit.

Es ist außerdem außerordentlich überraschend, daß erfindungsgemäß hochsubstituierte, filmbildende Galactomannane in einem Syntheseschritt synthetisiert werden können, die einerseits den Gastrointestinaltrakt unbeschadet überstehen, und andererseits im Dickdarm schnell abgebaut werden können. Dies war auch deshalb nicht zu erwarten, weil die Arbeitsgruppe um Isogai gezeigt hatte, daß veretherte Polysaccharide mit gesättigten aliphatischen Substituenten nur bis zu einem durchschnittlichen Substitutionsgrad von 2,5 erhalten werden können (Isogai, A., J. Appl. Polym. Sci. 29, 3873 - 3882 (1984)). Die Filmüberzüge aus den erfindungsgemäßen hochsubstituierten Ethylgalactomannanen können einerseits den Gastrointestinaltrakt unbeschadet überstehen. Andererseits werden sie im Dickdarm schnell abgebaut.

Dies war gerade deshalb überraschend, weil andere Arbeitsgruppen, beispielsweise Wrick M.G., J. Polym. SCI. A-1, 6, 1965 (1968) eine enzymatische Abbaubarkeit derivatisierter Polysaccharide widerlegten.

Die bisherigen bekannten niedrig substituierten Galactomannane besitzten die Eigenschaften, die die erfindungsgemäßen Galactomannane hinsichtlich Löslichkeit, Quellung und Abbaubarkeit aufweisen, insbesondere im Dickdarm, nicht.

Werden hingegen die vorliegenden Galactomannane zur Umhüllung oder Einbettung von Arzneimittelwirkstoffen verwendet, so erreichen die Arzneimittelwirkstoffe ohne Wirksamkeitsverlust den Dickdarm und werden erst dort freigegeben.

Die Erfindung betrifft ebenfalls ein Herstellungsverfahren zur Herstellung der neuen Galactomannanderivate entsprechend den Patentansprüchen.

Die Herstellung der erfindungsgemäß verwendeten Galactomannanether erfolgt dadurch, daß bekannte natürlich vorkommende Galactomannane oder synthetisch hergestellte Galactomannane mit Molekulargewichten zwischen 10⁴ und 10⁸ g in einem inerten Lösungsmittel (zum Beispiel Dimethylacetamid, Dimethylformamid, Dimethylsulfoxid) mit C₁-C₆-Alkylhalogeniden zum (Beispiel C₂-C₄-Alkylhalogeniden), Phenyl-C₁-C₄-Alkylhalogeniden oder Phenylhalogeniden bei Temperaturen zwischen 20 bis 35^{o}C, gegebenenfalls in Gegenwart von Alkali umgesetzt werden. Als Halogenide kommen vorzugsweise die Bromide in Frage. Es sind jedoch auch die Chloride und Jodide verwendbar. Alkali wird vorzugsweise in Form von Alkalihydroxiden, inbesondere NaOH, zugegeben.

Der Zusatz von Alkali hängt von der Größe des Ansatzes ab. Beispielsweise verwendet man bei einem Ansatz von 2,8 g bis 3,2 g Galactomannan 110 g bis 130 g Alkali (zum Beispiel in Form von NaOH).

Bei größeren Ansätzen benötigt man weniger Alkali (zum Beispiel 15 % weniger, bei kleineren Ansätzen benötigt man mehr Alkali (zum Beispiel 5 % bis 10 % mehr).

Es ist auch möglich mit dem zuvor angegebenen Verfahrens gemischte Ether zu bekommen, das heißt Ether die in demselben Molekül sowohl Alkylreste als auch Phenylalkylreste oder Phenylreste enthalten, indem man als Reaktionskomponente Mischungen der entsprechenden Halogenid-Ausgangssubstanzen verwendet.

Zur Herstellung der erfindungsgemäßen Ester muß wasserfrei gearbeitet werden. Als Lösungsmittel kommen hier in Frage:
Dimethylformamid, Dimethylacetamid, Dimethylsulfoxid. Die Veresterung kann auch ohne Lösungsmittel in der Schmelze durchgeführt werden. In diesem Falle dient als Lösungsmittel für das Polymer das Acylierungsreagens beziehungsweise der Reaktionspartner, zum Beispiel Alkaloylhalogenid oder Alkaloylanhydrid.

Die Reaktion der Veresterung erfolgt beispielsweise bei Temperaturen zwischen 70 bis 160^{o}C, beziehungsweise beim Siedepunkt des Lösungsmittels, durch Umsetzung der entsprechenden Galactomannane mit Molekulargewichten zwischen 10⁴ und 10⁸ g, mit C₃-C₂₂-Alkanoylhalogeniden, vorzugsweise C₃-C₁₆-Alkanoylhalogeniden oder C₃-C₂₂-Alkanoylanhydriden, vorzugsweise C₃-C₁₆-Alkanoylanhydriden.

Zweckmäßig erfolgt diese Acylierung in Gegenwart basischer Verbindungen, wie zum Beispiel Pyridin.

Die basischen Substanzen sollen bezogen auf die Ausgangsalkanoylverbindung im Überschuß vorliegen, beispielsweise im Überschuß von 0,1 bis 0,2 pro Mol der Alkanoylausgangskomponente.

Als Ausgangsgalactomannane werden beispielsweise natürlich vorkommende Galactomannane verwendet, beispielsweise Galactomannane aus Guarkernmehl, Tarakernmehl, Johannisbrotkernmehl.

Das Mannose-Galactoseverhältnis variiert je nach Herkunftspflanze von 1 : 1 bis etwa 7 : 1. Im allgemeinen liegt es beispielsweise zwischen 2 : 1 und 4 : 1 oder auch 2 : 1 bis 3 : 1. Es ist jedoch auch möglich das Mannose-Galactoseverhältnis dieser Ausgangsgalactomannane höher einzustellen indem man das Polysaccharid mit α-Galactosidase in hierfür bekannter Weise behandelt (siehe zum Beispiel Mc Cleary, B.V., Carbohyd. Res., 92, 269, (1981)

Zur Charakterisierung der Ausgangsgalactomannane dient neben dem Mannose-Galactoseverhältnis auch die Viskosität einer wäßrigen Lösung des Polysaccharids. Die Viskosität ist abhängig vom Molekulargewicht und demzufolge auch vom Vermahlungsgrad der galactomannanhaltigen Kerne. Das Molekulargewicht der Galactomannane kann je nach Herkunft und Meßmethode zwischen 10⁴ und 10⁸ g liegen. Durch Vermahlen kann man das Molekulargewicht der Polysaccharide reduzieren und auf einen erwünschten Bereich einstellen.

Der erwünschte Molekulargewichtsbereich ist vorzugsweise 10⁵ bis 10⁶.

Die im Handel befindlichen galactomannanhaltigen Mehle enthalten neben dem Polysaccharid auch etwa 5 % niedermolekulare Zucker, 1 % ölige Komponenten, 3 % Cellulose und 5 % Proteine. Daher muß das Galactomannan von diesen Begleitstoffen getrennt werden. Es ist also die vorschriftsmäßige Reinigung von Begleitstoffen, wie Proteinen und Lipiden, sowie die darauffolgende Fällung der Galactomannane mit anschlleßender Quellung des Polymeren, wie beispielsweise in dem Beispiel 1 beschrieben wird. Die in dem Beispiel 1 beschriebene Reinigung ist allgemein anwendbar, wobei lediglich die Zentrifugalkraft beim Zentrifugieren angepaßt werden muß. Wenn beispielsweise hochmolekulare oder mannosereichere Galactomannane vorliegen, sollte man bei entsprechend niedrigerer Geschwindigkeit zentrifugieren um auch mannosereichere Moleküle in Lösung zu halten. Die gereinigten Galactomannane werden auf Stickstoffgehalt geprüft. In einem proteinfreien Polysaccharid darf nach der Reinigung kein Stickstoff mehr nachweisbar sein.

Vorzugsweise haben die erfindungsgemäß verwendeten Galactomannanderivate keine freien Hydroxylgruppen mehr, beziehungsweise nur noch wenig freie Hydroxylgruppen, das heißt sie haben vorzugsweise einen statistischen Substitutionsgrad von 3. Im Durchschnitt sind 80 bis 100 % der vorhandenen freien Hydroxygruppen verethert und/oder verestert, vorzugsweise 90 bis 100 %.

Insbesondere betrifft die Erfindung die Verwendung der Galactomannanderivate zur Herstellung von Filmüberzügen und Einbettungen von pharmazeutischen Wirkstoffen, insbesondere von oral-applizierbaren Wirkstoffen, beziehungsweise oral-applizierbaren Arzneizubereitungen mit einer Wirkstoff-Freigabe im Dickdarm. Dies wird dadurch erreicht, daß die Wirkstoffe oder Zubereitungen mit Wirkstoffen, zum Beispiel Granulate, Pellets oder Tabletten, mit den erfindungsgemäßen Galactomannanderivaten umhüllt und/oder in diese eingebettet sind.

Die Darreichungsformen für die orale Anwendung enthalten bevorzugt 0,001 bis 500 mg Wirkstoff.

Die Erfindung betrifft auch ein Verfahren zur Herstellung von Arzneimittelzubereitungen, das dadurch gekennzeichnet ist, daß man einen Gewichtsteil Arzneimittelwirkstoff, wobei dieser auch in Form eines physiologisch verträglichen Salzes vorliegen kann, mit 10⁵ bis 10⁻² Gewichtsteilen Galactomannanen, deren Hydroxygruppen vollständig oder teilweise in Form niedrigmolekularer aliphatischer, araliphatischer und/oder aromatischer Ether- oder Estergruppen vorliegen und 20 bis 80 % der vorhandenen Mannose-Bausteine der Galactomannan-Kette zusammenhängende Mannose-Blöcke von 2 bis 20 Mannose Einheiten bilden, die nicht durch Galactose-Gruppen substituiert sind
a) in an sich bekannter Weise umhüllt oder
b) in an sich bekannter Weise in solche Galactomannane einbettet oder hieran bindet
wobei gegebenenfalls auch weitere übliche pharmazeutische Hilfs- und Zusatzstoffe mitverarbeitet werden können und gegebenenfalls die so erhaltenen Produkte zu Tabletten verpreßt oder in Kapseln abfüllt.

Die Umhüllung der Wirkstoffe oder der pharmazeutischen Zubereitungen, das heißt der Zubereitungen worin die Wirkstoffe zusammen mit üblichen oder erforderlichen pharmazeutischen Hilfsstoffen eingearbeitet sind, erfolgt nach den in der pharmazeutischen Technologie bekannten Methoden, beziehungsweise den üblichen Verfahren zum Überziehen von Arzneiformen. Das Einbetten von therapeutischen Wirkstoffen erfolgt ebenfalls nach in der pharmazeutischen Technologie bekannten Methoden. Es werden hierbei anstatt der bisher üblichen plastischen oder schmelzbaren Einbettungsmaterialien, zum Beispiel Wachse, hydriertes Ricinusöl, Kunststoffe, wie Celluloseether oder -ester, Poly(meth)acrylsäureester, die erfindungsgemäßen Galactomannanderivate verwendet. Hierbei können weiterhin übliche pharmazeutische Hilfs- beziehungsweise Zusatzstoffe mitverwendet werden, beispielsweise Weichmacher (insbesondere bei Umhüllungen), Aromastoffe, Süßmittel, Hilfsstoffe wie zum Beispiel Talkum, Calciumcarbonat, Mannitol, Cellulosepulver, lösliche Farbstoffe und Pigmente.

Die Hilfsstoffe werden, falls überhaupt, der Umhüllungsmischung beispielsweise in Mengen von 10 bis zu 100 Gewichts%, vorzugsweise von 20 bis zu 40 Gewichts%, bezogen auf das Gewicht der verwendeten Galactomannane, zugesetzt.

Die Aromastoffe, Süßmittel und Farbstoffe werden den Mischungen in geringen Mengen zugesetzt, beispielsweise von 0,001 % bis 2 %.

Nähere Angaben über die üblichen Hilfs- und Zusatzstoffe sind in der Fachliteratur, beispielsweise der Monographie von J.H. Saunders und K.C. Frisch "High Polymers", Verlag Interscience Publishers 1962 beziehungsweise 1964, zu entnehmen.

Zur Umhüllung von Arzneimittelwirkstoffen oder Arzneizubereitungen mit den erfindungsgemäßen Galactomannan-Derivaten.

Hier können beispielsweise zusätzlich auch übliche Weichmacher (z.B. Dibutylsebacat, Citronen- und Weinsäureester, Glycerin und Glycerinester, Phthalsäureester und ähnliche Stoffe) verwendet werden, außerdem ist der Zusatz wasserlöslicher Substanzen wie Polyethylenglykole, Polyvinylpyrrolidon, Copolymerisat aus Polyvinylpyrrolidon und Polyvinylacetat, Hydroxypropylcellulose, Hydroxypropylmethylcellulose möglich. Auch der Zusatz von Feststoffen wie Talkum und/oder Magnesiumstearat in der Umhüllung oder in Tablettenmischungen ist möglich.

Die Umhüllung erfolgt durch Aufsprühen von Lösungen in organischen Lösungsmitteln oder Suspensionen beziehungsweise Dispersionen der genannten Substanzen in organischen Lösungsmitteln oder Wasser, wobei noch weitere Hilfsstoffe zur Optimierung deren Verarbeitbarkeit zugesetzt sein können, wie zum Beispiel oberflächenaktive Substanzen, Pigmente.

Das Aufsprühen erfolgt zum Beispiel im Dragierkessel oder in perforierten Kesseln, oder im Luftsuspensionsbeziehungsweise Wirbelschichtverfahren (zum Beispiel Glatt Wirbelschichtanlage WLSD5).
Die Umhüllung kann auch im Koazervationsverfahren erfolgen, wobei sogenannte Mikrokapseln oder Mikropartikel gebildet werden.

Die Umhüllung kann auch durch Koagulation wässriger Dispersionen oder Suspensionen der zuvor genannten Substanzen erfolgen, indem der Wirkstoff mit der Dispersion vermischt und das Wasser durch Trocknen entfernt wird.

Überzogene Wirkstoffteilchen und überzogene Granulate können zu Tabletten verpreßt werden, überzogene Pellets in Hartgelatinekapseln abgefüllt werden.

Beim Überziehen von Wirkstoffteilchen oder Granulaten, die Wirkstoffteilchen enthalten, wird üblicherweise mehr Umhüllungsmaterial eingesetzt als bei Pellets oder Tabletten, da die Oberfläche, die bedeckt werden muß, wesentlich größer ist als bei Pellets oder Tabletten.

Da Tabletten in der Regel größer als Pellets sind, ist die zu bedeckende Oberfläche bei Tabletten entsprechend kleiner.

Auf 1 Gewichtsteil Wirkstoff oder Arzneizubereitung können 0,02 bis 0,5 Gewichtsteile Galactomannan-Derivat als Hüllmaterial verwendet werden. Bevorzugt ist ein Gewichtsverhältnis von 1 Teil Wirkstoff und 0,04 bis 0,3 Gewichtsteilen Hüllmaterial, ganz besonders bevorzugt sind 0,05 bis 0,2 Gewichtsteile Hüllmaterial pro 1 Gewichtsteil Wirkstoff. Das Aufbringen des Hüllmaterials in Lösung, Suspension beziehunsweise Dispersion erfolgt bei erhöhter Temperatur, vorzugsweise im Luftstrom. Zulufttemperatur zum Beispiel 70 bis 90^{o}C; Temperatur der Abluft zum Beispiel bis 40^{o}C.

Zur Einbettung der Wirkstoffe oder Bindung an die erfindungsgemäßen Galactomannan-Derivate.

Auf 1 Gewichtsteil Wirkstoff werden hier beispielsweise 0,05 bis 5,0 Gewichtsteile Galactomannan-Derivat, vorzugsweise 0,08 bis 3,0 Gewichtsteile, ganz besonders bevorzugt 0,1 bis 2,0 Gewichtsteile verwendet. Die Herstellung dieser Zubereitungen erfolgt bei Temperaturen zwischen 10^{o}C und 100^{o}C.

Die Herstellung dieser Darreichungsformen kann zum Beispiel erfolgen:
a) durch Lösen oder Dispergieren der Wirkstoffe oder deren Salzen in den erfindungsgemäßen Galactomannan-Derivaten oder Mischungen hiervon, auch unter Schmelzen der genannten Substanzen und anschließend Wiederabkühlen, Zerkleinern, eventuell Zufügen weiterer Substanzen wie zum Beispiel wasserlöslichen oder in Wasser quellbaren Substanzen und Verpressung zu Tabletten. Das Abkühlen der Schmelze und Zerkleinern kann auch in einem Schritt zusammengefaßt werden, indem die Schmelze in kaltem Wasser dispergiert wird oder einer Spüherstarrung unterworfen wird.
   Als Quellstoffe kommen zum Beispiel in Frage: Methylcellulose, Hydroxypropylcellulose, Hydroxypropylmethylcellulose (Pharmacoat, Methocel E (Cellulosemischether mit Propoxy- und Methoxysubstituenten), Alginsäure und ihre Salze (Na-, Ca-Salz, auch Mischungen aus Natriumalginat und Calciumsalzen zum Beispiel CaHPO₄), Stärke, Carboxymethylstärke, Carboxymethylcellulose und deren Salze (zum Beispiel Na-Salz), Gummi arabicum, Karaya Gummi, Ghatti Gum, Agar-agar, Carrageen, Xanthan-Gummi, Propylenglykolalginat, Pektin, Traganth.
b) durch Mischen der Wirkstoffe mit den erfindungsgemäßen Galactomannan-Derivaten und gegebenenfalls Quellstoffen oder Mischungen dieser Substanzen, auch unter Anwendung von Wärme, und beispielsweise Verpressen der Mischungen, eventuell nach Zusatz weiterer Hilfsstoffe, zu Tabletten oder Formung zu Pellets sowie Granulaten.
c) durch Mischen der Wirkstoffe mit Lösungen der erfindungsgemäßen Galactomannan-Derivate in organischen Lösungsmitteln, wie zum Beispiel Ethanol, Ethylacetat, Aceton oder Isopropanol, eventuell Mischen mit Trägermaterialien wie Cellulosen, sowie nachfolgendes Abdampfen der Lösungsmittel und Vermischen der erhaltenen Wirkstoffeinbettung mit weiteren Hilfsstoffen und Verarbeitung zu Formkörpern, wie zum Beispiel Tabletten, Granulaten oder Pellets.
d) durch Anfeuchten einer Mischung der Wirkstoffe und der erfindungsgemäßen Galactomannane gegebenenfalls den genannten Quellstoffen mit organischen Lösungsmitteln wie Ethanol, Ethylacetat, Aceton oder Isopropanol, eventuell unter Beifügung von Bindemitteln, wie Polyvinylpyrrolidon oder Copolymeren aus Polyvinylpyrrolidon und Polyvinylacetat, Granulieren der erhaltenen Mischung, nachfolgendes Trocknen, Zusatz von eventuellen weiteren Hilfsstoffen und beispielsweise Pressung der Mischung zu Tabletten,
e) durch Mischen der Wirkstoffe mit einer Lösung der erfindungsgemäßen Galactomannan-Derivate in Polyethylenglykol vom Molgewicht 200 bis 1500, eventuell Zusatz weiterer Hilfsstoffe, wie zum Beispiel Stearate oder Quellstoffe und beispielsweise Verkapseln der erhaltenen Masse in Weich- oder Hartgelatinekapseln.

Ganz allgemein erfolgt die Herstellung dieser Arzneimittelzubereitungen in an sich bekannter Weise, wobei neben den erfindungsgemäßen Galactomannan-Derivaten die bekannten und üblichen pharmazeutischen Hilfsstoffe sowie sonstige übliche Träger- und Verdünnungsmittel verwendet werden können.
Als derartige Träger- und Verdünnungsmittel kommen zum Beispiel auch solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 und ff., H.V.Czetsch-Lindenwald, Hilfsstoffe für Pharmazie und angrenzende Gebiete; Pharm. Ind., Heft 2, 1961, Seite 72 und ff.; Dr. H.P.Fiedler, Lexikon der Hilfsstoffe für Pharmazie, Kosmetik und angrenzende Gebiete, 2. Auflage, Editio Cantor, Aulendorf in Württemberg 1981.

Beispiele für übliche Hilfsstoffe, Trägerstoffe und Verdünnungsmittel sind Gelatine, natürliche Zucker, wie Rohrzucker oder Milchzucker, Lecithin, Pektin, Stärke (z.B. Maisstärke) sowie Stärkederivate, Cyclodextrine und Cyclodextrinderivate, Polyvinylpyrrolidon, Gelatine, Gummi arabicum, Alginsäure, Tylose, Talkum, Kieselsäure (zum Beispiel kolloidale) beziehungsweise hochdisperses SiO₂, Lävulose, Traganth, Natriumchlorid, Stearate, Magnesium- und Calciumsalze von Fettsäuren mit 12 bis 22 C-Atomen, insbesondere der gesättigten (zum Beispiel Stearate), Polyethylenglycol mit einem mittleren Molekulargewicht zwischen 200 und 20 000, vorzugsweise zwischen 200 und 5 000, insbesondere zwischen 200 und 1 000, oder deren Gemische und/oder Polymerisate aus Vinylpyrrolidon und/oder Mischpolymerisate aus Vinylpyrrolidon und Vinylacetat, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglycol, Pentaerythrit, Sorbit, Mannit usw. die gegebenenfalls auch verethert sein können, Benzylbenzoat, Dioxolane, Glyzerinformale, Tetrahydrofurfurylalkohol, Polyglykolether mit C₁ bis C₁₂-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat, Gummi arabicum, Alginsäure, Stearate, Fette und ähnlich wirkende Stoffe.

Daneben können die Darreichungsformen grenzflächenaktive Substanzen enthalten. Als Beispiele seien genannt: Alkaliseifen, wie Alkalisalze höherer Fettsäuren (zum Beispiel Na-palmitat, Na-stearat) oder deren Derivate (zum Beispiel Na-rizinolatschwefelsäureester); sulfurierte Verbindungen oder sulfonierte Verbindungen, die durch Umsetzung von höheren Fettalkoholen mit Schwefelsäure oder Chlorsulfonsäure entstehen und zum Beispiel als Natriumsalze eingesetzt werden (zum Beispiel Natriumlaurylsulfat, Natriumcetylsulfat, Natriumstearylsulfat, Natriumcetylsulfonat); Salze der Gallensäuren; Saponine; quartäre Ammoniumverbindungen; Partialfettsäureester des Sorbitans; Partialfettsäureester und Fettsäureester des Polyoxyethylensorbitans; Sorbitolether des Polyoxyethylens; Fettsäureester des Polyoxyethylens; Fettalkoholether des Polyoxyethylens; Fettsäureester der Saccharose; Fettsäureester des Polyglycerols; Proteine; Lecithine.

Die Darreichungsformen können auch Cellulosen enthalten, insbesondere wenn Komprimate hergestellt werden sollen. Als solche kommen in Frage:
gereinigte Cellulose (zum Beispiel als Elcema^{®} im Handel) oder mikrokristalline Cellulose, wie sie zum Beispiel unter dem Namen Avicel^{®} im Handel ist. Aber auch andere Füllmittel wie Calciumhydrogenphosphat, Milchzucker, Stärken (zum Beispiel Kartoffelstärke, Maisstärke), Glucose, Mannit und Saccharose, sowie Füllmittel mit Bindemittelfunktion, wie mikrokristalline Cellulose, hydrolysierte oder teilabgebaute Stärken und Mischkristallisate aus Cellulosepulver und Lactose.

Die Darreichungsformen können darüber hinaus Sedimentationsverzögerer enthalten, wie zum Beispiel hochdisperse Kieselsäuren, die eine Oberfläche von 50 bis 500 m²/g, insbesondere 100 bis 400 m²/g aufweisen (bestimmt nach der BET-Methode). Diese sind im Handel zum Beispiel unter dem Namen Aerosil^{®} erhältlich.

Außerdem kann der Einsatz von Formtrennmitteln in der Darreichungsform sinnvoll sein. Als solche wären zu nennen:
Talk oder sllikonisierter Talk, Calcium- und Magnesiumstearat, Stearinsäure, Paraffin, hydrierte Fette und Öle, Siliconölemulsion.

Als weitere Hilfsstoffe kommen auch Stoffe in Frage, die den Zerfall bewirken (sogenannte Sprengmittel) wie:
quervernetztes Polyvinylpyrrolidon, Natriumcarboxymethylstärke, Natriumcarboxymethylcellulose, Formaldehydgelatine, Formaldehydcasein, Polyacrylsäure, Ultraamylopektin.

Darüberhinaus ist der Zusatz von Stabilisatoren, Farbstoffen, Antioxidantien und Komplexbildnern (zum Beispiel Ethylendiaminotetraessigsäure) und dergleichen möglich sowie den Zusatz von Säuren wie Citronensäure, Weinsäure, Maleinsäure, Fumarsäure.

Als Antioxidantien kommen beispielsweise Natriummetabisulfit, Cystein, Ascorbinsäure und deren Ester (zum Beispiel -palmitat), Flavonoide, Gallussäure-alkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Citronensäure, Phophorsäure) zur Anwendung.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Als Plastifizierungsmittel für die erfindungsgemäßen Galactomannan-Derivate können in Frage kommen:
Citronen- und Weinsäureester (Acetyltriethyl-, Acetyltributyl-, Tributyl-, Triethyl-citrat); Glycerin und Glycerinester (Glycerindiacetat, -triacetat, acetylierte Monoglyceride, Rizinusöl); Phthalsäureester (Dibutyl-, Diamyl-, Diethyl-, Dimethyl-, Dipropyl-, D-(2-Methoxy- oder ethoxyethyl)-phthalat, Ethylphthalyl- und Butylphthalylethyl- und butylglycolat); Alkohole (Propylenglykol, Polyethylenglykol verschiedener Kettenlängen), Adipate (Diethyl-, Di(2-Methoxy- oder ethoxyethyl-adipat); Benzophenon; Diethyl- und Dibutylsebacat, -succinat, -tartrat; Diethylenglykoldipropionat; Ethylenglykoldiacetat, -dibutyrat, -dipropionat; Tributylphosphat, Tributyrin; Polyethylenglykolsorbitanmonooleat; Sorbitanmonooleat.

Zum Aufbringen der erfindungsgemäßen Galactomannan-Derivate können Lösungsmittel verwendet werden aus der Gruppe der wässrigen Lösungsmittel, Alkohole, Ketone, Ester, Ether, aliphatischen Kohlenstoffe, halogenierten Lösungsmittel, cycloaliphatischen, heterocyclischen Lösungsmitteln und deren Gemische. Typische Lösungsmittel sind unter anderem Aceton, Diaceton-alkohol, Methanol, Ethanol, Isopropylalkohol, Butylalkohol, Methylacetat, Ethylacetat, Isopropylacetat, n-Butylacetat, Methylisobutyl-keton, Methyl-propyl-keton, n-Hexan, n-Heptan, Ethylglykol-monoethylether, Ethylenglykol-monoethylacetat, Methylendichlorid, Ethylendichlorid, Propylendichlorid, Tetrachlorkohlenstoff, Nitroethan, Nitropropan, Tetrachlorethan, Ethylether, Isopropylether, Cyclohexan, Cyclooctan, Benzol, Toluol, Naphtha, 1,4-Dioxan, Tetrahydrofuran, Diethylenglykoldimethylether, Wasser und deren Gemische wie Aceton und Wasser, Aceton und Methanol, Aceton und Ethylalkohol, Methylendichlorid und Methanol und Ethylendichlorid und Methanol sowie deren Gemische. Im Laufe des Umhüllungsprozesses werden diese Lösungsmittel wieder entfernt.

Als Wirkstoffe, die vorzugsweise mit den erfindungsgemäßen Galactomannan-Derivaten forumuliert werden können, kommen beispielsweise insbesondere solche Arzneimittelwirkstoffe in Frage, die im Dünndarm verdaut werden und deshalb peroral nicht appliziert werden können, vorzugsweise Peptid-Arzneimittel. Beispiele sind: Peptide, Herz-Kreislauf-Therapeutika, Antirheumatika/Analgetika, Mittel zur Therapie von Dickdarmerkrankungen (Morbus Crohin, Colitis Ulcerosa), Antiasthmatika, Antifibrinolytika, Antihämorrhagika, Antitumormittel, Enzympräparate, Antibiotika, Antimykotika, Substanzen mit Wirkung auf das ZNS (Zentralnervensystem).

Beispiele für Peptid-Wirkstoffe sind insbesondere:
ACTH (Adrenocorticotropes Hormon), Corticostatin, Calcitonin, Insulin, Oxytocin, Somatostatin und Analoga, LHRH-Analoga, Bombesin-Analoga, Cholecystokinin und Derivate, Endothelin und Analoga, Thrombin-Inhibitoren, Peptide Growth Factors (zum Beispiel IGF, EGF, NGF) Magainine (PGS peptides), Gastrin-Analoga, Bradykinin-Analoga, Parathormon Analoga, Neurokinin und Analoga, VIP und Analoga, ANP (Atriales Natriuretisches Peptid) und Analoga, Neokyotrophin und Analoga, Angiotensin-Analoga, Enkephaline, Dynorphine, Dermorphine, Deltorphine, Renin-inhibierende Peptide, Tumor-Growth-Factor-Peptide, MSH (Melanocyte Stimulating Hormone)-Analoga, Mitotoxine, Tyrphostine, Chromogranin A, Thymopentin, TRH und Analoga, Substanz-P, Tuftsin, Fibronectin, und peptidische Immunmodulatoren wie Cyclosporin A, FK 506, Neuropeptid Y und NPK.

Bei Arzneiformen, die in Gegenwart von glykolytischen Enzymen den Wirkstoff freisetzen, handelt es sich vorzugsweise um umhüllte feste Arzneizubereitungen, die mit den erfindungsgemäßen Galactomannan-Derivaten umhüllt sind, wie zum Beispiel Dragees, Kapseln, Filmtabletten, Pellets oder Mikropartikel. Hierbei werden die Arzneimittelwirkstoffe freigesetzt im Dickdarm nach oraler Gabe durch glykolytischen Abbau des Polymers.

Als glykolytische Enzyme kommen hierbei die folgenden in Frage: Mannasen, Mannosidasen, Galactosidasen, wie zum Beispiel β-D-Mannanase, β-D-Mannosidase, α-D-Mannosidase, β-D-Galactanase, β-D-Galactosidase, α-D-Galactosidase, β-D-Glucosidase und α-D-Glucosidase, β-D-Glucuronidase, α-L-Arabinosidase, β-D-Fucosidase, β-D-Xylanase, β-D-Xylosidase, Pectinase.

Gegebenenfalls ist es auch möglich, den erfindungsgemäßen Arzneimittelzubereitungen getrennt solche glykolytischen Enzyme zuzusetzen, wobei die Formulierung dann so zu wählen ist, daß am Wirkungsort diese glykolytischen Enzyme freigesetzt werden. Dies kann beispielsweise durch folgende Ausführungen geschehen:

2-Kompartiment-Formulierungen zur Einbringung in die Blase oder andere Körperhöhlen enthalten den Wirkstoff, zum Beispiel ein Antibiotikum, in einem mit den erfindungsgemäßen Galactomannanen überzogenen Kompartiment, während in das 2. Kompartiment glykolytische Enzyme eingebracht werden. Durch Überzug des 2. Kompartiments mit säure- oder alkalilöslichen Filmen, etwa vom Typ der Polyacrylate (zum Beispiel Eudragite), läßt sich die Aktivierung der Enzyme und nachfolgend der Freisetzung des Wirkstoffs in Abhängigkeit vom pH-Wert der Umgebung dergestalt steuern, daß der Polyacrylatfilm nur in einem bestimmten pH-Bereich für das zur Enzymaktivierung notwendige Wasser permeabel wird.

### Beispiel 1

### Galactomannan-methylether (Substitutionsgrad 3,0)

Eingesetzt wird gereinigtes, proteinfreies Galactomannan, gewonnen aus Johannisbrotkernmehl (locust bean gum) mit dem Vermahlungsgrad 175.

50 g Johannisbrotkernmehl (Vidogum L 175, Unipectin AG, Eschenz/Schweiz) wird in einem 5 Liter Erlenmeyerkolben mit 70 g Methanol befeuchtet, um eine klumpenfreie Lösung mit Wasser herzustellen. Mit abgekochtem destilliertem Wasser wird auf 5 Liter aufgefüllt und über Nacht gerührt.

Anschließend wird die Suspension bei 6500 g etwa 20 Minuten lang zentrifugiert. Die überstehende Lösung wird von den Proteinen abdekantiert. Jeweils 2,5 Liter dieser Lösung werden mit demselben Volumen Methanol versetzt und geschüttelt. Das rein weiß ausfallende Galactomannan wird abfiltriert und mehrmals mit Methanol ausgewaschen.
Das gereinigte Polymer wird im Kühlschrank, in Methanol suspendiert, verschlossen aufbewahrt. Das Polymer wird vom Methanol abfiltriert und in einen 1 Liter Birnenkolben auf 100 g eingewogen.

Eine weitere Probe von 5 g wird im Trockenschrank bei 120^{o}C getrocknet und gewogen, um die in der Synthese eingesetzte Trockenmasse an Galactomannan zu berechnen. Diese soll zwischen 2,8 g und 3,2 g betragen.

Nach Zugabe von 215 ml Dimethylsulfoxid wird die Suspension am Rotationsverdampfer mit Stickstoff begast, und unter Wasserstrahlvakuum bei 45°C das Methanol abrotiert. Der so gewonnene Polymerbrei wird unter Stickstoffbegasung in einen 2 Liter Braunglas-Dreihalskolben mit KPG-Rührer* und Intensivkühler (Temperatur 5°C) überführt.
* Rührer, der durch einen Elektromotor angetrieben wird und durch einen eingeschliffenen Verschluß in das Rührgefäß eingeführt wird.

Nach 15 Minuten werden 120 g Natriumhydroxid unter Rühren zugegeben. Unmittelbar danach werden die restlichen 200 ml Dimethylsulfoxid zugegeben. Dieser Ansatz wird eine Stunde gerührt. Anschließend versetzt man den Poymerbrei mit 626 ml Jodmethan. Die vorübergehend entstehende geringe Wärme (30°C) wird nicht abgeführt. Über die restliche Reaktionszeit bleibt eine Reaktionstemperatur von 20°C bis 25°C gewährleistet.

Nach 48 Stunden läßt man das Reaktionsgemisch absitzen und dekantiert den Überstand in einen 1 Liter Braunglasbirnenkolben.

Der Inhalt dieses Kolbens wird am Rotationsdampfer mit Stickstoff belüftet und am Wasserstrahlvakuum bei 65°C abgezogen bis kein Lösungsmittel mehr übertritt. Sodann wird am Öldrehschiebervakuum bei 65°C das restliche Dimethylsulfoxid über eine Kühlfalle (flüssiger Stickstoff) abgezogen.

Der abrotierte Rückstand wird gesammelt und mit 1 bis 2 Liter peroxidfreiem Diethylether versetzt. Die Suspension wird in einen 2 Liter Dreihalskolben gegeben und über Nacht unter Rückfluß bei Raumtemperatur gerührt.

Der Etherextrakt wird sodann abgenutscht und der Filterrückstand mehrmals mit Diethylether ausgewaschen. Die vereinigten Filtrate werden am Rotationsverdampfer unter Wasserstrahl vakuum bei 40°C abgezogen bis eine Phasentrennung stattfindet und sich das Polymer fast rein abscheidet. Die verbleibende gelbe Dimethylsulfoxid-Phase wird vom Polymer abdekantiert.

Das Polymer wird auf Filterpapier abgepreßt und in Methanol gelöst. Die konzentrierte, hochviskose Methanollösung (ca. 100 ml) wird in 5 Liter heißes Wasser (60°C) unter Rühren eingegeben. Das ausgefallene Polymer wird im Trockenschrank bei 60°C auf einer Petrischale erhitzt, bis sich das Polymer vom Wasser getrennt hat.

Sodann wird das Polymer auf Filterpapier abgepreßt und im Trockenschrank bei 120°C getrocknet. Nach dem Wiegen wird das Polymer in Chloroform gelöst und in Petrischalen ausgegossen. Diese werden bis zur Filmbildung bei Raumtemperatur staubgeschützt trocknen gelassen. Man erhält sehr zugfeste, elastische Filme, die klar und farblos sind.

Wird die Synthese im größeren Maßstab durchgeführt (zum Beispiel unter Verwendung von mehr als 12 g Galactomannan), wird die Abfuhr der Reaktionswärme beim Start der Reaktion zunehmend wichtiger. Eine Aufwärmung des Reaktionsgemisches über 35°C ist unerwünscht, weil sonst gelbe Nebenprodukte entstehen. Diese Nebenprodukte färben das Ethylgalactomannan stark an und sind sehr schwer zu entfernen. Darum soll bei größeren Ansätzen das Reaktionsgemisch mit Hilfe eines Wasserbades auf 20°C gekühlt und stark gerührt werden.

Um eine Ausbeute über 80 % zu erreichen muß das gesamte Reaktionsgemisch ständig durchmischt werden. Bei Synthesen in kleineren Maßstäben genügt ein KPG-Rührer bei mittleren Drehzahlen. Bei größeren Ansätzen aber besteht die Gefahr, daß entweder die Edukte zu wenig durchgemischt werden oder, daß die suspendierten Galactomannanfäden sich um den Rührstab herumwickeln und nicht reagieren. Darum ist es nötig aufwendigere Rührwerke einzusetzen. Setzt man nur einen großflügeligen Rührer ein, so ergibt sich außen am Rührflügel eine große Umfangsgeschwindigkeit und eine zu hohe Scherkraft.

### Beispiel 2

Galactomannanethylether (Substitutionsgrad 3,0)

Eingesetzt wird gereinigtes, proteinfreies Galactomannan, gewonnen aus Johannisbrotkernmehl (locust bean gum) mit dem Vermahlungsgrad 175.

50 g Johannisbrotkernmehl (Vidogum L 175, Unipectin AG, Eschenz/Schweiz) wird in einem 5 Liter Erlenmeyerkolben mit 70 g Methanol befeuchtet, um eine klumpenfreie Lösung mit Wasser herzustellen. Mit abgekochtem detilliertem Wasser wird auf 5 Liter aufgefüllt und über Nacht gerührt.

Anschließend wird die Suspension bei 6500 g etwa 20 Minuten lang zentrifugiert. Die überstehende Lösung wird von den Proteinen abdekantiert. Jeweils 2,5 Liter dieser Lösung werden mit demselben Volumen Methanol versetzt und geschüttelt. Das rein weiß ausfallende Galactomannan wird abfiltriert und mehrmals mit Methanol ausgewaschen.
Das gereinigte Polymer wird im Kühlschrank, in Methanol suspendiert, verschlossen aufbewahrt. Das Polymer wird vom Methanol abfiltriert und in einen 1 Liter Birnenkolben auf 100 g eingewogen.

Eine weitere Probe von 5 g im Trockenschrank bei 120°C getrocknet und gewogen, um die in der Synthese eingesetzte Trockenmasse an Galactomannan zu berechnen. Diese soll zwischen 2,8 g und 3,2 g betragen.

Nach Zugabe von 215 ml Dimethylsulfoxid wird die Suspension am Rotationsverdampfer mit Stickstoff begast, und unter Wasserstrahlvakuum bei 45°C das Methanol abrotiert. Der so gewonnene Polymerbrei wird unter Stickstoffbegasung mit KPG-Rührer* und Intensivkühler (Temperatur 5°C) überführt.
* Rührer, der durch einen Elektromotor angetrieben wird und durch einen eingeschliffenen Verschluß in das Rührgefäß eingeführt wird.

Nach 15 Minuten werden 120 g Natriumhydroxid unter Rühren zugegeben. Unmittelbar danach werden die restlichen 200 ml Dimethylsulfoxid zugegeben. Dieser Ansatz wird eine Stunde gerührt. Anschließend versetzt man den Polymerbrei mit 745 ml Bromethan. Die vorübergehend entstehende geringe Wärme (30°C) wird nicht abgeführt. Über die restliche Reaktionszeit bleibt eine Reaktionstemperatur von 20°C bis 25°C gewährleistet.

Nach 48 Stunden läßt man das Reaktionsgemisch absitzen und dekantiert den Überstand in einen 1 Liter Braunglasbirnenkolben.

Der Inhalt dieses Kolbens wird am Rotationsdampfer mit Stickstoff belüftet und am Wasserstrahl vakuum bei 65°C abgezogen bis kein Lösungsmittel mehr übertritt. Sodann wird am Öldrehschiebervakuum bei 65°C das restliche Dimethylsulfoxid über eine Kühlfalle (flüssiger Stickstoff) abgezogen.

Der abrotierte Rückstand wird gesammelt und mit 1 bis 2 Liter peroxidfreiem Diethylether versetzt. Die Suspension wird in einen 2 Liter Dreihalskolben gegeben und über Nacht unter Rückfluß bei Raumtemperatur gerührt.

Der Etherextrakt wird sodann angenutscht und der Filterrückstand mehrmals mit Diethylether ausgewaschen. Die vereinigten Filtrate werden am Rotationsverdampfer unter Wasserstrahl vakuum bei 40°C abgezogen bis eine Phasentrennung stattfindet und sich das Polymer fast rein abscheidet. Die verbleibende gelbe Dimethylsulfoxid-Phase wird vom Polymer abdekantiert.

Das Polymer wird auf Filterpapier abgepreßt und in Methanol gelöst. Die konzentrierte, hochviskose Methanollösung (ca. 100 ml) wird in 5 Liter heißes Wasser (60°C) unter Rühren eingegeben. Das ausgefallende Polymer wird im Trockenschrank bei 60°C auf einer Petrischale erhitzt bis sich das Polymer vom Wasser getrennt hat.

Sodann wird das Polymer auf Filterpapier abgepreßt und im Trockenschrank bei 120°C getrocknet. Nach dem Wiegen wird das Polymer in Chloroform gelöst und in Petrischalen ausgegossen. Diese werden bis zur Filmbildung bei Raumtemperatur staubgeschützt trocknen gelassen. Man erhält sehr zugfeste, elastische Filme, die klar und farblos sind.

Wird die Synthese im größeren Maßstab durchgeführt (zum Beispiel unter Verwendung von mehr als 12 g Galactomannan), wird die Abfuhr der Reakionswärme beim Start der Reaktion zunehmend wichtiger. Eine Aufwärmung des Reakionsgemisches über 35°C ist unerwünscht, weil sonst gelbe Nebenprodukte entstehen. Diese Nebenprodukte färben das Ethylgalactomannan stark an und sind sehr schwer zu entfernen. Darum soll bei größeren Ansätzen das Reakionsgemisch mit Hilfe eines Wasserbades auf 20°C gekühlt und stark gerührt werden.

Um eine Ausbeute über 80 % zu erreichen muß das gesamte Reaktionsgemisch ständig durchmischt werden. Bei Synthesen in kleineren Maßstäben genügt ein KPG-Rührer bei mittleren Drehzahlen. Bei größeren Ansätzen aber besteht die Gefahr, daß entweder die Edukte zu wenig durchgemischt werden oder, daß die suspendierten Galactomannanfäden sich um den Rührstab herumwickeln und nicht reagieren. Darum ist es nötig aufwendigere Rührwerke einzusetzen. Setzt man nur einen großflügeligen Rührer ein, so ergibt sich außen am Rührflügel eine große Umfangsgeschwindigkeit und eine zu hohe Scherkraft.

### Beispiel 3

### Acetylgalactomannanethylether (Mischsubstitution)

Eingesetzt wird gereinigtes, proteinfreies Galactomannan, gewonnen aus Johannisbrotkernmehl (locust bean gum) mit dem Vermahlungsgrad 175.

50 g Johannisbrotkernmehl (Vidogum L 175, Unipectin AG, Eschenz/Schweiz) wird in einem 5 Liter Erlenmeyerkolben mit 70 g Methanol befeuchtet, um eine klumpenfreie Lösung mit Wasser herzustellen. Mit abgekochtem detilliertem Wasser wird auf 5 Liter aufgefüllt und über Nacht gerührt.

Anschließend wird die Suspension bei 6500 g etwa 20 Minuten lang zentrifugiert. Die überstehende Lösung wird von den Proteinen abdekantiert. Jeweils 2,5 Liter dieser Lösung werden mit demselben Volumen Methanol versetzt und geschüttelt. Das rein weiß ausfallende Galactomannan wird abfiltriert und mehrmals mit Methanol ausgewaschen.
Das gereinigte Polymer wird im Kühlschrank, in Methanol suspendiert, verschlossen aufbewahrt. Das Polymer wird vom Methanol abfiltriert und in einen 1 Liter Birnenkolben auf 100 g eingewogen.

Eine weitere Probe von 5 g im Trockenschrank bei 120°C getrocknet und gewogen, um die in der Synthese eingesetzte Trockenmasse an Galactomannan zu berechnen. Diese soll zwischen 2,8 g und 3,2 g betragen.

Nach Zugabe von 215 ml Dimethylsulfoxid wird die Suspension am Rotationsverdampfer mit Stickstoff begast, und unter Wasserstrahlvakuum bei 45°C das Methanol abrotiert. Der so gewonnene Polymerbrei wird unter Stickstoffbegasung mit KPG-Rührer* und Intensivkühler (Temperatur 5°C) überführt.
* Rührer, der durch einen Elektromotor angetrieben wird und durch einen eingeschliffenen Verschluß in das Rührgefäß eingeführt wird.

Nach 15 Minuten werden 120 g Natriumhydroxid unter Rühren zugegeben. Unmittelbar danach werden die restlichen 200 ml Dimethylsulfoxid zugegeben. Dieser Ansatz wird eine Stunde gerührt. Anschließend versetzt man den Polymerbrei mit 745 ml Bromethan. Die vorübergehend entstehende geringe Wärme (30°C) wird nicht abgeführt. Über die restliche Reaktionszeit bleibt eine Reaktionstemperatur von 20°C bis 25°C gewährleistet.

Nach 2 Stunden läßt man das Reaktionsgemisch absitzen und dekantiert den Überstand in einen 1 Liter Braunglasbirnenkolben.

Der Inhalt dieses Kolbens wird am Rotationsdampfer mit Stickstoff belüftet und am Wasserstrahlvakuum bei 65^{o}C abgezogen bis kein Lösungsmittel mehr übertritt. Sodann wird am Öldrehschiebervakuum bei 65^{o}C das restliche Dimethylsulfoxid über eine Kühlfalle (flüssiger Stickstoff) abgezogen.

Der abrotierte Rückstand wird gesammelt und mit 1 bis 2 Liter peroxidfreiem Diethylether versetzt. Die Suspension wird in einen 2 Liter Dreihalskolben gegeben und über Nacht unter Rückfluß bei Raumtemperatur gerührt.

Der Etherextrakt wird sodann abgenutscht und der Filterrückstand mehrmals mit Diethylether ausgewaschen. Die vereinigten Filtrate werden am Rotationsverdampfer unter Wasserstrahlvakuum bei 40^{o}C abgezogen bis eine Phasentrennung stattfindet und sich das Polymer fast rein abscheidet. Die verbleibende gelbe Dimethylsulfoxid-Phase wird vom Polymer abdekantiert.

Das Polymer wird auf Filterpapier abgepreßt und in Methanol gelöst. Die konzentrierte, hochviskose Methanollösung (ca. 100 ml) wird in 5 Liter heißes Wasser (60^{o}C) unter Rühren eingegeben. Das ausgefallene Polymer wird im Trockenschrank bei 60^{o}C auf einer Petrischale erhitzt, bis sich das Polymer vom Wasser getrennt hat.

Sodann wird das Polymer auf Filterpapier abgepreßt und im Trockenschrank bei 120^{o}C getrocknet.

Das Polymer wird in 250 ml Acetanhydrid gelöst und in einen 1-Liter Rundkolben mit Rückflusskühler und Magnetrührer überführt. Nach Zusatz von 300 bis 350 ml Pyridin wird 5 Stunden lang bis zum Sieden unter Rühren erhitzt.

Danach wird die heiße Reaktionslösung in 8 bis 10 l Wasser gegeben und das gefällte Polymer in Methanol gewaschen. Das abgepresste Polymer wird dann in wenig Chloroform gelöst und erneut in 5 l Methanol gefällt.

Sodann wird das Polymer im Trockenschrank bei 80^{o}C getrocknet. Nach dem Wiegen wird das Polymer in Chloroform gelöst und in Petrischalen ausgegossen. Diese werden bis zur Filmbildung bei Raumtemperatur staubgeschützt trocknen gelassen. Man erhält sehr zugfeste, elastische Filme, die klar und farblos sind.

## Patentansprüche

1. Verwendung von Galactomannanen, deren Hydroxygruppen vollständig oder teilweise in Form niedrigmolekularer aliphatischer, araliphatischer und/oder aromatischer Ether- oder Estergruppen vorliegen, und 20 bis 80 % der vorhandenen Mannose-Bausteine der Galactomannan-Kette zusammenhängende Mannose-Blöcke von 2 bis 20 Mannose Einheiten bilden, die nicht durch Galactose-Gruppen substituiert sind, für Umhüllungen und/oder Einbettungen von festen und/oder flüssigen Wirkstoffen, welche diese Wirkstoffe erst im Dickdarm oder in Gegenwart von glykolytischen Enzymen freigeben oder zur Herstellung von Folien.

2. Verwendung nach Anspruch 1, dadurch gekennzeichnet, daß die Ethergruppen aus C₁-C₆-Alkoxygruppen, Phenyl-C₁-C₄-alkoxygruppen oder Phenoxygruppen und die Estergruppen aus C₃-C₂2-Alkanoyloxygruppen bestehen.

3. Verwendung nach einem oder mehreren der vorargegengenen Ansprüche, dadurch gekennzeichnet, daß das Molgewicht der erfindungsgemäßen Galactomannane zwischen 10⁴ und 10⁸ insbesondere 10⁵ und 10⁶ liegt.

4. Verwendung nach einem oder mehreren der vorangegangenen Ansprüche, dadurch gekennzeichnet, daß das Mannose,Galactose-Verhältnis der zugrundeliegenden Galactomannan-Kette zwischen 2:1 und 20:1 vorzugsweise 4:1 liegt.

5. Verwendung nach einem oder mehreren der vorangegangenen Ansprüche, dadurch gekennzeichnet, daß im Durchschnitt 80 bis 100 % der vorhandenen freien Hydroxygruppen des zugrunde liegenden Galactomannans verethert und/oder verestert sind.

6. Arzneimittelzubereitung dadurch gekennzeichnet, daß Arzneimittelwirkstoffe oder deren physiologisch verträglichen Salze gegebenenfalls unter Zusatz von sonstigen üblichen Hilfs- und Zusatzstoffen
a) mit einem oder mehreren cder erfindungsgemäßen Galactomannan-Derivate nach Anspruch 1 umhüllt sind oder
b) in eine oder mehrere der erfindungsgemäßen Galactomannan-Derivate nach Anspruch 1 eingebettet oder hieran gebunden sind.

7. Arzneimittelzubereitung nach Anspruch 6, dadurch gekennzeichnet, daß die Darreichungsformen für die orale Anwendung 0,001 bis 500 mg Wirkstoff enthalten.

8. Verfahren zur Herstellung von Arzneimittelzubereitungen, dadurch gekennzeichnet, daß man einen Gewichtsteil Arzneimittelwirkstoff, wobei dieser auch in Form eines physiologisch verträglichen Salzes vorliegen kann, mit 10⁵ bis 10⁻² Gewichtsteilen Glactomannanen, deren Hydroxygruppen vollständig oder teilweise in Form niedrigmolekularer aliphatischer, araliphatischer und/oder aromatischer Ether- oder Estergruppen vorliegen und 20 bis 80 % der vorhandenen Mannose-Bausteine der Galactomannan-Kette zusammenhängende Mannose-Blöcke von 2 bis 20 Mannose Einheiten bilden, die nicht nur Galactose-Gruppen substituiert sind
a) in an sich bekannter Weise umhüllt oder
b) in an sich bekannter Weise in solche Galactomannane einbettet oder hieran bindet
wobei gegebenenfalls auch weitere übliche pharmazeutische Hilfs- und Zusatzstoffe mitverarbeitet werden können und gegebenenfalls die so erhaltenen Produkte zu Tabletten verpreßt oder in Kapseln abfüllt.

9. Verfahren nach Anspruch 8, dadurch gekennzeichnet, daß die Umhüllung der Wirkstoffe durch Aufsprühen oder Koagulation unter Verwendung von Lösungen, Suspensionen oder Dispersionen der erfindungsgemäßen Galactomannane in organischen Mitteln oder Wasser erfolgt.

10. Verfahren nach Anspruch 8, dadurch gekennzeichnet, daß die Einbettung der Wirkstoffe erfolgt
a) durch Lösen oder Dispergieren der Wirkstoffe in den erfindungsgemäßen Galactomannanen
b) durch Mischen der Wirkstoffe mit den erfindungsgemäßen Galactomannanen
c) durch Mischen der Wirkstoffe mit Lösungen der erfindungsgemäßen Galactomannane in organischen Mitteln
d) durch Anfeuchten einer Mischung der Wirkstoffe und der erfindungsgemäßen Galactomannane, gegebenenfalls in Gegenwart von Quellstoffen in organischen Lösungsmitteln und anschließendem Granulieren
e) durch Mischen der Wirkstoffe mit einer Lösung der erfindungsgemäßen Galactomannane in Polyethylenglykolen und gegebenenfalls anschließendem Abfüllen des erhaltenen Produktes in Kapseln.

## Claims

1. The use of galactomannans whose hydroxyl groups are completely or partially in the form of low molecular weight aliphatic, araliphalic and/or aromatic ether or ester groups, and 20 to 80% of the mannose units present in the galactomannan chain form continuous mannose blocks of 2 to 20 mannose units which are not substituted by galactose groups, for enveloping and/or embedding solid and/or liquid active substances, which release these active substances only in the large intestine or in the presence of glycolytic enzymes, or for producing sheets.

2. The use as claimed in claim 1, wherein the ether groups consist of C₁-C₆-alkoxy groups, phenyl-C₁-C₄-alkoxy groups or phenoxy groups and the ester groups consist of C₃-C₂₂-alkanoyloxy groups.

3. The use as claimed in one or more of the preceding claims, wherein the molecular weight of the galactomannans according to the invention is from 10⁴ to 10⁸, in particular 10⁵ to 10⁶.

4. The use as claimed in one or more of the preceding claims, wherein the mannose/galactose ratio in the underlying galactomannan chain is from 2:1 and 20:1, preferably 4:1.

5. The use as claimed in one or more of the preceding claims, wherein on average 80 to 100% of the free hydroxyl groups present in the underlying galactomannan are etherified and/or esterified.

6. A pharmaceutical composition wherein pharmaceutical active substances or their physiologically tolerated salts are, where appropriate with the addition of other customary ancillary substances and additives,
a) enveloped with one or more of the galactomannan derivatives according to the invention as set forth in claim 1, or
b) embedded in one or more of the galactomannan derivatives according to the invention as set forth in claim 1, or bound thereto.

7. A pharmaceutical composition as claimed in claim 6, wherein the administration forms for oral use contain from 0.001 to 500 mg of active substance.

8. A process for the production of pharmaceutical compositions, which comprises one part by weight of pharmaceutical active substance, which can also be in the form of a physiologically tolerated salt, being, with the use of 10⁵ to 10⁻² parts by weight of galactomannans whose hydroxyl groups are completely or partially in the form of low molecular weight aliphatic, araliphatic and/or aromatic ether or ester groups, and 20 to 80% of the mannose units present in the galactomannan chain form continuous mannose blocks of 2 to 20 mannose units which are not substituted by galactose groups,
a) enveloped in a conventional way or
b) embedded in such galactomannans, or bound thereto, in a conventional way,
it being possible also to use other conventional pharmaceutical ancillary substances and additives where appropriate, and the products obtained in this way being, where appropriate, compressed to tablets or filled into capsules.

9. A process as claimed in claim 8, wherein the enveloping of the active substances takes place by spraying on or coagulation using solutions, suspensions or dispersions of the galactomannans according to the invention in organic agents or water.

10. A process as claimed in claim 8, wherein the embedding of the active substances takes place
a) by dissolving or dispersing the active substances in the galactomannans according to the invention
b) by mixing the active substances with the galactomannans according to the invention,
c) by mixing the active substances with solutions of the galactomannans according to the invention in organic agents
d) by moistening a mixture of the active substances and of the galactomannans according to the invention, where appropriate in the presence of swelling agents in organic solvents and subsequently granulating
e) by mixing the active substances with a solution of the galactomannans according to the invention in polyethylene glycols and, where appropriate, subsequently filling the resulting product into capsules.

## Revendications

1. Utilisation de galactomannanes dont les groupes hydroxy sont en totalité ou en partie à l'état de groupes éther ou ester aliphatiques, araliphatiques et/ou aromatiques à bas poids moléculaire, et 20 à 80 % des motifs de mannose présentent dans la chaîne de galactomannane forment des blocs cohérents de mannose comportant 2 à 20 motifs de mannose qui ne sont pas substitués par des groupes galactose, pour des enrobages et/ou des noyages de substances actives solides et/ou liquides, ne libérant ces substances actives que dans le gros intestin ou en présence d'enzymes glycolytiques, ou pour la fabrication de feuilles.

2. Utilisation selon la revendication 1, caractérisée par le fait que les groupes éther consistent en groupes alcoxy en C1-C6, phénylalcoxy en C1-C4 ou phénoxy, et les groupes ester consistent en groupes alcanoyloxy en C3-C22.

3. Utilisation selon une ou plusieurs des revendications qui précédent, caractérisée par le fait que le poids moléculaire des galactomannanes selon l'invention se situe entre 10⁴ et 10⁸, plus spécialement entre 10⁵ et 10⁶.

4. Utilisation selon une ou plusieurs des revendications qui précédent, caractérisée par le fait que, dans la chaîne de galactomannane les contenant, le rapport mannose/galactose se situe entre 2 : 1 et 20 : 1 et est de préférence de 4 : 1.

5. Utilisation selon une ou plusieurs des revendications qui précèdent, caractérisée par le fait que 80 à 100 % en moyenne des groupes hydroxy libres de chaîne de galactomannane sont éthérifiés et/ou estérifiés.

6. Médicament, caractérisé par le fait que les substances actives médicamenteuses ou leurs sels acceptables pour l'usage pharmaceutique, avec le cas échéant des produits auxiliaires et additifs usuels
a) sont enrobées par un ou plusieurs des dérivés de galactomannane selon l'invention selon la revendication 1, ou bien
b) sont noyées dans un ou plusieurs des dérivés de galactomannanes selon l'invention de la revendication 1, ou fixées sur ceux-ci.

7. Médicament selon la revendication 6, caractérisé par le fait que les formes d'administration orales contiennent de 0,001 à 500 mg de substance active.

8. Procédé de préparation de médicaments caractérisé par le fait que
a) on enrobe de manière connue en soi, ou bien
b) on noie ou on fixe de manière connue en soi
une partie en poids de la substance active médicamenteuse, qui peut également être à l'état de sel acceptable pour l'usage pharmaceutique, dans 10⁵ à 10⁻² parties en poids d'un galactomannane dont les groupes hydroxy sont en totalité ou en partie à l'état de groupes éther ou ester aliphatiques, araliphatiques et/ou aromatiques à bas poids moléculaire et 20 à 80 % des motifs de mannose présents dans la chaîne de galactomannane forment des blocs cohérents de mannose comportant 2 à 20 motifs de mannose qui ne sont pas substitués par des groupes galactose,
et le cas échéant, on combine d'autres produits auxiliaires et additifs pharmaceutiques usuels et le cas échéant on met les produits obtenus à l'état de comprimés à la presse ou on les introduit dans des capsules.

9. Procédé selon la revendication 8, caractérisé par le fait que l'enrobage des substances actives est réalisé par pulvérisation ou coagulation à l'aide de solutions, suspensions ou dispersions des galactomannanes selon l'invention dans des liquides organiques ou de l'eau.

10. Procédé selon la revendication 8, caractérisé par le fait que le noyage des substances actives est réalisé
a) par dissolution ou dispersion des substances actives dans les galactomannanes selon l'invention,
b) par mélange des substances actives avec les galactomannanes selon l'invention,
c) par mélange des substances actives avec des solutions des galactomannanes selon l'invention dans des milieux organiques,
d) par humidification d'un mélange des substances actives et des galactomannanes selon l'invention, le cas échéant en présence d'agents gonflants, dans des solvants organiques, en faisant suivre d'une granulation,
e) par mélange des substances actives avec une solution des galactomannanes selon l'invention dans des polyéthylèneglycols et, le cas échéant, introduction subséquente du produit obtenu dans des capsules.
